# EUROPÄISCHE PATENTSCHRIFT

(11) **EP 2 404 595 B1**
(45) Veröffentlichungstag und Bekanntmachung des Hinweises auf die Patenterteilung: **05.08.2015**
(21) Anmeldenummer: 11179791.6
(22) Anmeldetag: 01.09.2011
(51) Int. Cl.: C07C 45/74, C07C 49/755, C07C 49/683, A61K 31/122, A61K 31/35, A61Q 17/04, A61Q 19/00, C07C 45/72, A61K 8/35

(54) **Verfahren zur Herstellung von Indanon-Derivaten**
Method for manufacturing indanone derivatives
Procédé destiné à la fabrication de dérivés d'indanone

(43) Veröffentlichungstag der Anmeldung: 11.01.2012
(73) Patentinhaber: Symrise AG, 37603 Holzminden (DE)
(72) Erfinder: Koch, Oskar, 37079 Göttingen (DE); Schatkowski, Dietmar, 37574 Einbeck (DE); Johncock, William, 21465 Reinbek (DE); Jahnke, Bodo, 33034 Brakel (DE); Fahlbusch, Karl-Georg, 37671 Höxter (DE)
(74) Vertreter: Eisenführ Speiser

(56) Entgegenhaltungen:
- WO-A1-2007/128723
- IMBACH J-L ET AL: "NUCLEAR MAGNETIC RESONANCE SPECTRA OF DERIVATIVES OF VARIOUS SUBSTITUTED INDANONES AND TETRALONES", TETRAHEDRON, ELSEVIER SCIENCE PUBLISHERS, AMSTERDAM, NL, Bd. 23, 1. Januar 1967 (1967-01-01), Seiten 3931-3941, XP001544619, ISSN: 0040-4020, DOI: 10.1016/S0040-4020(01)97903-7
- DATABASE WPI Week 199225 Thomson Scientific, London, GB; AN 1992-204486 XP002667500, & JP 4 134043 A (KAO CORP) 7. Mai 1992 (1992-05-07)

## Beschreibung

Die vorliegende Erfindung betrifft ein Verfahren zur Herstellung einer Verbindung der Formel (I) 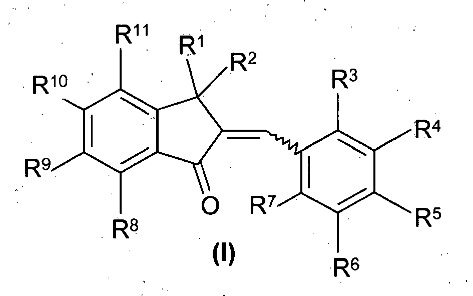
wobei R¹ und R² unabhängig voneinander jeweils Wasserstoff oder eine verzweigte oder unverzweigte Alkylgruppe mit 1 bis 12 Kohlenstoffatomen bedeuten und
wobei R³, R⁴, R⁵, R⁶, R⁷, R⁸, R⁹, R¹⁰ und R¹¹ unabhängig voneinander jeweils Wasserstoff, Hydroxy oder eine verzweigte oder unverzweigte Alkyl- oder Alkoxygruppe mit 1 bis 12 Kohlenstoffatomen bedeuten.

Unter den vorstehend angegebenen Verbindungen der Formel (I) besitzt die Verbindung eine besondere Bedeutung, bei der R¹ und R² jeweils Methyl bedeuten, R⁹ und R¹⁰ jeweils Methoxy bedeuten und R³, R⁴, R⁵, R⁶, R⁷, R⁸ und R¹¹ jeweils Wasserstoff bedeuten. Diese Verbindung ist 2-Benzyliden-3,3-dimethyl-5,6-dimethoxy-1-indanon (CAS Nr. 924656-15-3; INCI Bezeichnung: Benzylidene Dimethoxydimethylindanone).

Spezifische Verbindungen der Formel (I) sowie die im Rahmen der vorliegenden Erfindung besonders relevante Verbindung 2-Benzyliden-3,3-dimethyl-5,6-dimethoxy-1-indanon sind aus der Veröffentlichung WO 2007/128723 A1 (Symrise) bekannt. Sie sind als Aryl-Hydrocarbon-Rezeptor-Antagonisten (AhRezeptor-Antagonist; AhR-Antagonist) wirksam. Spezielle Wirkungen, Verwendungen und Rezepturen (Formulierungen, Zubereitungen etc.) umfassend beziehungsweise betreffend eine Verbindung der Formel (I) ergeben sich aus der WO 2007/128723 A1.

Die durch das erfindungsgemäße Verfahren hergestellten Verbindungen der Formel (I), insbesondere die bevorzugte Verbindung 2-Benzyliden-3,3-dimethyl-5,6-dimethoxy-1-indanon, liegen als E- oder Z-Isomer oder in Form einer E/Z-Isomerenmischung vor. Im vorliegenden Text wird in Formelbildern eine geschlängelte Linie verwendet um anzuzeigen, dass die entsprechende Verbindung E- oder Z-konfiguriert ist oder eine Mischung von E- und Z-Isomeren vorliegt. Die Veröffentlichung WO 2007/128723 A1 offenbart in Beispiel 1 ein Verfahren zur Herstellung von 2-Benzyliden-3,3-dimethyl-5,6-dimethoxy-1-indanon. Die Reaktionsgleichung für die Synthesereaktion ist nachfolgend angegeben:

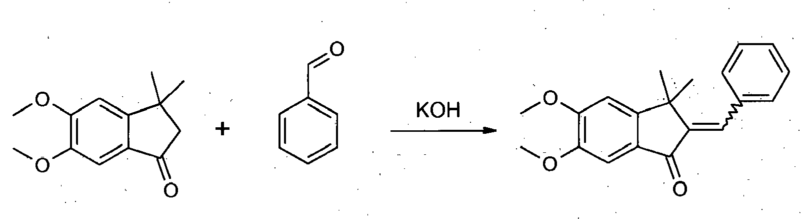

Gemäß Beispiel 1 der WO 2007/128723 A1 wird die Verbindung 3,3-Dimethy-5,6-dimethoxy-1-indanon (erster Reaktant gemäß vorstehender Reaktionsgleichung) zu einer Suspension von Kaliumhydroxid in Diethylenglykoldimethylether hinzugefügt. Nach Erhitzen auf 80°C wird Benzaldehyd im Verlauf von einer Stunde zudosiert und die Mischung für weitere 3h bei der besagten Temperatur gerührt. Hierbei wird das 3,3-Dimethyl-5,6-dimethoxy-1-indanon mit dem Benzaldehyd umgesetzt. Anschließend wird auf Raumtemperatur abgekühlt, Eiswasser wird hinzugegeben und durch Zugabe von Salzsäure neutralisiert. Nach Extraktion mit Methyl-tert-butylether wird das Produkt aus Methanol umkristallisiert. Es wird ein E/Z-Isomerengemisch in einer Ausbeute von 76 % d. Th. erhalten. Die gemäß Beispiel 1 der WO2007/128723 A1 eingesetzte Menge an Kaliumhydroxid entspricht ca. 40 Mol-% bezogen auf die eingesetzte Menge an 3,3-Dimethyl-5,6-dimethoxy-1-indanon. Bei eigenen Nacharbeitungen zur Verfahrensgestaltung gemäß WO2007/128723 A1 konnte nach Kristallisation bestenfalls ein Produkt mit einem Anteil von 99,6 % des Zielproduktes 2-Benzyliden-3,3-dimethyl-5,6-dimethoxy-1-indanon und 0,3 % 2-Benzyl-3,3-dimethyl-5,6-dimethoxy-1-indanon (als Nebenprodukt) erhalten werden, welches stark gelb gefärbt war. Es sei insoweit darauf hingewiesen, dass nach der Zugabe von Wasser, wie sie gemäß WO2007/128723 A1 vorgesehen ist, ein weiteres, nicht mit Wasser mischbares Lösungsmittel zugesetzt werden muss, um das Produkt aus der wässrigen Phase zu extrahieren. Dieser Extraktionsschritt ist mit Aufwand und Kosten verbunden.

Das in WO 2007/128723 A1 offenbarte Verfahren zur Herstellung von 2-Benzyliden-3,3-dimethyl-5,6-dimethoxy-1-indanon ist zwar praxistauglich, besitzt jedoch noch eine Reihe von wirtschaftlichen und ökologischen Nachteilen. So wird gemäß WO 2007/128723 A1 das Edukt-Indanon in Diethylenglykoldimethylether gelöst; diese Verbindung ist jedoch gemäß des Global harmonisierten Systems zur Einstufung und Kennzeichnung von Chemikalien (GHS) ein sogenannter CMR-Stoff (canzerogen, mutagen, reproduktionstoxisch) der Kategorie 1B (es existieren hinreichende Anhaltspunkte für CMR-Eigenschaften). Dies ist gerade für Substanzen, die im kosmetischen Bereich Anwendung finden sollen, kritisch beziehungsweise inakzeptabel, da nicht mit ausreichender Sicherheit ausgeschlossen werden kann, dass Restmengen an Diethylenglykoldimethylether im Produkt (mit dem Hauptbestandteil 2-Benzyliden-3,3-dimethyl-5,6-dimethoxy-1-indanon) verbleiben. Zudem ist der Diethylenglykoldimethylether sehr gut wasserlöslich, so dass er mit dem im Produktgemisch vorhandenen Wasser ins Abwasser gelangt. Die Aufbereitung des Abwassers oder dessen Entsorgung sind wirtschaftlich nachteilig, zudem sind die Kosten für den Diethylenglykoldimethylether selbst beträchtlich, insbesondere, da es nach der Reaktion nicht in ausreichend reiner Form zurückgewonnen werden kann. Insgesamt erscheint daher das in der WO 2007/128723 A1 offenbarte Verfahren unwirtschaftlich.

Zudem entsteht bei der Verfahrensgestaltung gemäß WO 2007/128723 A1 als unerwünschtes Nebenprodukt die Verbindung 2-Benzyl-3,3-dimethyl-5,6-dimethoxy-1-indanon in einer Konzentration, die in den meisten Fällen bis zu ca. 0,5 Gew.-% beträgt, in manchen Fällen jedoch auch darüber liegt. Die Verbindung 2-Benzyl-3,3-dimethyl-5,6-dimethoxy-1-indanon besitzt eine Struktur gemäß der nachfolgenden Formel (V):

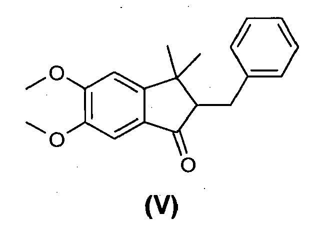

Das Nebenprodukt (V) unterscheidet sich vom Zielprodukt (2-Benzyliden-3,3-dimethyl-5,6-dimethoxy-1-indanon) somit lediglich dadurch, dass es keine Doppelbindung in 2-Position des 5-Rings besitzt. Das Nebenprodukt kann durch übliche physikalische Trennverfahren wie Destillation oder Kristallisation nicht hinreichend vom Zielprodukt abgetrennt werden, so dass dieses bei der Verfahrensgestaltung gemäß WO2007/128723 A1 stets mit erheblichen Mengen des Nebenprodukts verunreinigt ist. Im Unterschied zum Zielprodukt 2-Benzyliden-3,3-dimethyl-5,6-dimethoxy-1-indanon ist das Nebenprodukt 2-Benzyl-3,3-dimethyl-5,6-dimethoxy-1-indanon in kosmetischen Ölen schlecht löslich. In eigenen Untersuchungen hat sich gezeigt, dass bereits ein Anteil von 250 ppm (0,025 Gew.-%) des Nebenproduktes (V) bezogen auf die Gesamtmasse einer üblichen kosmetischen Formulierung, die Bildung von Kristallisationskeimen initiiert. Vergleiche hierzu die Beispiele weiter unten. Da üblicherweise jedoch an kosmetische Formulierungen die Anforderung gestellt wird, dass sie homogen sein müssen, ist die Anwesenheit von Formulierungsbestandteilen mit schlechter Löslichkeit in kosmetischen Ölen regelmäßig nicht akzeptabel. Die Anwesenheit des Nebenprodukts ist somit ein weiterer Nachteil, der mit der Verfahrensgestaltung gemäß WO 2007/128723 A1 verbunden ist.

Schließlich besitzt das gemäß Beispiel 1 der WO 2007/128723 A1 hergestellte Produkt beziehungsweise Produktgemisch eine störende Gelbfärbung, die auf das Vorhandensein (strukturell derzeit noch nicht identifizierter) weiterer Nebenprodukte intensiver Farbe schließen lässt. Die gelbe Färbung des Produktes beziehungsweise der Produktgemische gemäß WO 2007/128723 A1 hat einen nachteiligen Einfluss auf die Farbe eines jeden kosmetischen oder pharmazeutischen Produktes, welches unter Zusatz des gemäß WO 2007/128723 A1 hergestellten Produktes hergestellt wird. Die gelbe Färbung des besagten Produktes ist somit ein weiterer erheblicher Nachteil, der mit der Durchführung des Verfahrens gemäß WO 2007/128723 A1 verbunden ist.

Schließlich ist darauf hinzuweisen, dass gemäß der Verfahrensgestaltung aus WO2007/128723 A1 hohe Mengen an Kaliumhydroxid (als Katalysatorbase) zugesetzt werden (ca. 40 Mol-%, bezogen auf die eingesetzte Menge des Indanon-Derivats), was bei der Aufarbeitung des Reaktionsgemisches durch Neutralstellen mit Salzsäure zu erheblichen Salzfrachten im Abwasser führt. Dies ist ein weiterer ökologischer Nachteil.

Im Zusammenhang mit der vorliegenden Erfindung ist auch auf IMBACH J-L ET AL: "NUCLEAR MAGNETIC RESONANCE SPECTRA OF DERIVATIVES OF VARIOUS SÜBSTITUTED INDANONES AND TETRALONES", TETRAHEDRON, ELSEVIER SCIENCE PUBLISHERS, AMSTERDAM, D2, NL, Bd. 23, 1. Januar 1967 (1967-01-01), Seiten 3931-3941, und JP 4 134043 A zu verweisen. Darin werden (u.a.) Verfahren offenbart, bei denen Ethanol, welches bei 20 °C sehr gut wasserlöslich ist, als Lösungsmittel verwendet wird.

Es war die Aufgabe der vorliegenden Erfindung, ein Verfahren anzugeben, welches zumindest einige, vorzugsweise jedoch sämtliche der vorstehend genannten Nachteile der Verfahrensgestaltung gemäß WO 2007/128723 A1 lindert oder vorzugsweise komplett beseitigt. Bevorzugte anzugebende Verfahren sollten somit möglichst zu Produktgemischen führen, welche nicht durch Diethylenglykoldimethylether verunreinigt sind, wobei die resultierenden Produkte vorzugsweise eine reduzierte Menge des unerwünschten Nebenproduktes der Formel (IV) 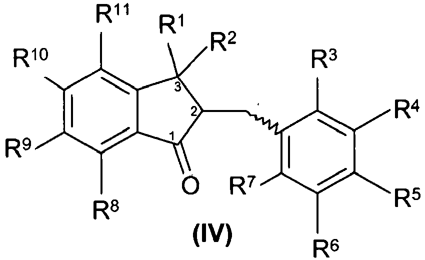 umfassen sollten sowie vorzugsweise eine reduzierte Menge der strukturell nicht identifizierten Verunreinigungen enthalten sollten, welche die gelbe Färbung von Produkten gemäß WO 2007/128723 A1 verursachen. Das Nebenprodukt der Formel (IV) unterscheidet sich von der Verbindung der Formel (I) dadurch, dass keine Doppelbindung in 2-Position des 5-Rings vorhanden ist (vgl. die Nummerierung im 5-Ring der Formel (IV)). Weitere Aufgabenstellungen ergeben sich aus der nachfolgenden Beschreibung. Erfindungsgemäß werden zumindest einzelne der vorstehend genannten Aufgaben gelöst durch ein Verfahren zur Herstellung einer Verbindung der Formel (I), 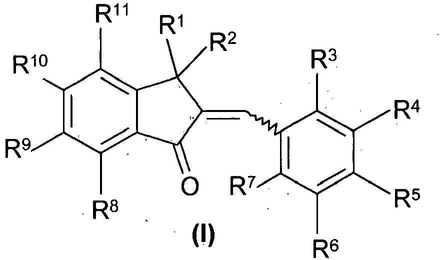 oder einer Mischung umfassend eine Verbindung der Formel (I), mit folgenden Schritten:
- Lösen eines Indanons der Formel (II) in einem Lösungsmittel oder in einer ein Lösungsmittel enthaltenden Mischung, wobei das Lösungsmittel ausgewählt ist aus der Gruppe der Lösungsmittel, die mit Wasser ein Azeotrop bilden,
- Umsetzen des Indanons der Formel (II) mit einem aromatischen Aldehyd der Formel (III) in Gegenwart zumindest einer organischen Base, gemäß dem Schema 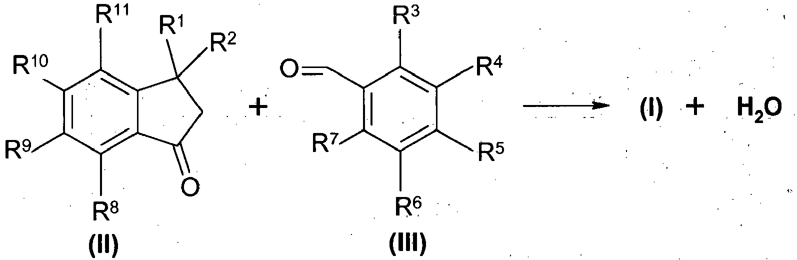
   wobei R¹ und R² unabhängig voneinander jeweils Wasserstoff oder eine verzweigte oder unverzweigte Alkylgruppe mit 1 bis 12 Kohlenstoffatomen bedeuten und
   wobei R³, R⁴, R⁵, R⁶, R⁷, R⁸, R⁹, R¹⁰ und R¹¹ unabhängig voneinander jeweils Wasserstoff, Hydroxy oder eine verzweigte oder unverzweigte Alkyl- oder Alkoxygruppe mit 1 bis 12 Kohlenstoffatomen bedeuten und
- Entfernen von gebildetem Wasser aus dem Reaktionsgemisch während der Umsetzung.

Es versteht sich, dass die Substituenten R¹ bis R¹¹ in Formel (I) immer genau die Bedeutungen besitzen, die sie in den Formeln (II), (III) bzw. (IV) besitzen.

Vorzugsweise bedeuten in der Verbindung der Formel (I) sowie den Edukt-Verbindungen der Formeln (II) und (III) die Substituenten R¹ und R² unabhängig voneinander jeweils Methyl, Ethyl, n-Propyl oder iso-Propyl und die Substituenten R³, R⁴, R⁵, R⁶, R⁷, R⁸, R⁹, R¹⁰ und R¹¹ bedeuten unabhängig voneinander jeweils Wasserstoff, Methyl, Ethyl, n-Propyl, iso-Propyl, Methoxy, Ethoxy, Propoxy oder Butoxy.

Besonders bevorzugt bedeuten in den besagten Formeln die Substituenten R¹ und R² jeweils Methyl und die Substituenten R³, R⁶, R⁷, R⁸ und R¹¹ jeweils Wasserstoff und die Substituenten R⁴, R⁵, R⁹ und R¹⁰ unabhängig voneinander jeweils Wasserstoff, Methoxy oder n-Propoxy.

Ganz besonders bevorzugt bedeuten die Substituenten R¹ und R² jeweils Methyl, die Substituenten R⁹ und R¹⁰ jeweils Methoxy und die Substituenten R³, R⁴, R⁵, R⁶, R⁷, R⁸ und R¹¹ jeweils Wasserstoff. Wie bereits erwähnt, liegt ein Schwerpunkt der vorliegenden Erfindung auf einem Verfahren zur Herstellung von 2-Benzyliden-3,3-dimethyl-5,6-dimethoxy-1-indanon. Diese Verbindung ist eine solche Verbindung der Formel (I), in der gemäß der vorstehend als besonders bevorzugt bezeichneten Ausgestaltung R¹ und R² jeweils Methyl bedeuten, R⁹ und R¹⁰ jeweils Methoxy bedeuten und R³, R⁴, R⁵, R⁶, R⁷, R⁸ und R¹¹ jeweils Wasserstoff bedeuten.

Die allgemeine Reaktionsgleichung betreffend die Bildung von 2-Benzyliden-3,3-dimethyl-5,6-dimethoxy-1-indanon ist nachfolgend angegeben:

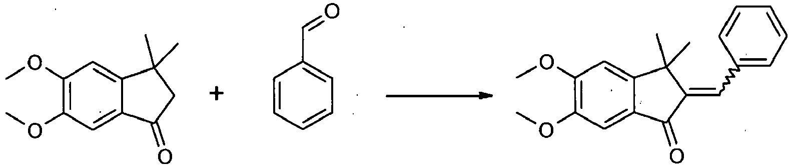

Erfindungsgemäß wird im Unterschied zu der Verfahrensgestaltung gemäß WO2007/128723 A1 während der Umsetzung das durch den Reaktionsschritt der Eliminierung gebildete Wasser aus dem Reaktionsgemisch entfernt. Überraschenderweise hat sich in eigenen Untersuchungen gezeigt, dass sich bei einer solchen erfindungsgemäßen Verfahrensgestaltung bei Einsatz von Lösungsmitteln, die mit Wasser ein Azeotrop bilden und bei Einsatz einer organischen Base als Katalysator nicht nur die Ausbeute der Synthesereaktion im Vergleich zur Verfahrensgestaltung gemäß WO2007/128723 A1 deutlich erhöhen lässt, sondern auch der Gehalt an Nebenprodukten (Benzyl-Derivate; gelbfärbende Substanzen) deutlich reduziert wird. Der Gehalt an als Nebenprodukt auftretenden Benzyl-Derivaten war in eigenen Untersuchungen regelmäßig so gering, dass er in Routine-GC-Untersuchungen nicht mehr detektierbar war.

Vorzugsweise wird in einem erfindungsgemäßen Verfahren die organische Base so ausgewählt, dass sie das Indanon der Formel (II) zur Umsetzung mit dem Aldehyd der Formel (III) in das entsprechende Enolat überführt. Dies bedeutet, dass die vorzugsweise eingesetzte organische Base selbst als Katalysator für die stattfindenden Teilreaktionen (Aldol-Addition und Aldol-Kondensation) fungiert.

Vorzugsweise ist die organische Base ein Alkoholat, vorzugsweise ein Alkalimetall- oder Erdalkalimetallalkoholat, vorzugsweise ein Alkalimetall- oder Erdalkalimetallalkoholat mit verzweigtem Alkoholatanion.

Besonders bevorzugt ist die organische Base ausgewählt aus der Gruppe bestehend aus Kalium-*tert*-butanolat, Natrium-*tert*-butanolat, Lithium-*tert*-butanolat, Barium-*tert*-butanolat, Magnesium-*tert*-butanolat, Kalium-*tert*-pentanolat, Natrium-*tert-*pentanolat und Lithium-*tert*-pentanolat, wobei die organische Base bevorzugt ausgewählt ist aus der Gruppe bestehend aus Natrium-*tert*-butanolat und Kalium-*tert-*butanolat.

Bei Einsatz der besonders bevorzugten organischen Basen hat sich in eigenen Untersuchungen gezeigt, dass besonders gute Ausbeuten am jeweiligen Zielprodukt erreicht werden konnten, wobei gleichzeitig die Menge gebildeter Nebenprodukte drastisch reduziert war.

Zudem kann bei Einsatz der als besonders bevorzugt bezeichneten organischen Basen eine vergleichsweise geringe Menge dieser Basen eingesetzt werden, was dazu beiträgt, die oben geschilderten Nachteile zu lindern, die mit der Entstehung der entsprechenden Salze verbunden sind.

Wie vorstehend bereits angemerkt, ist insbesondere bei Einsatz einer vorstehend als bevorzugt bezeichneten organischen Base eine Verfahrensgestaltung möglich, bei der die eingesetzte Menge der organischen Base vergleichsweise gering ist. Vorteilhafterweise ist das Stoffmengenverhältnis der eingesetzten Menge von organischer Base zur eingesetzten Menge von Indanon der Formel (II) kleiner als 0,50, vorzugsweise kleiner als 0,20, bevorzugt kleiner als 0,10, besonders bevorzugt kleiner 0,05.

Selbstverständlich wird in einem erfindungsgemäßen Verfahren jedoch üblicherweise eine katalytisch wirksame Menge der organischen Base eingesetzt, wobei es sich bei der organischen Base vorzugsweise um eine der oben genannten bevorzugten Basen handelt. In eigenen Untersuchungen haben sich Verfahrensgestaltungen als besonders effektiv erwiesen, in denen das Stoffmengenverhältnis der eingesetzten Menge von organischer Base zur eingesetzten Menge von Indanon der Formel (II) größer als 0,005, vorzugsweise größer als 0,01, bevorzugt größer als 0,02 ist. Dabei gilt weiterhin, dass das Stoffmengenverhältnis der eingesetzten Menge von organischer Base zur eingesetzten Menge von Indanon der Formel (II) kleiner als 0,50, vorzugsweise kleiner als 0,20, bevorzugt kleiner als 0,10, besonders bevorzugt kleiner 0,05 ist.

Durch Einsatz einer entsprechenden Menge an organischer Base lässt sich die Menge an Salz reduzieren, die ansonsten durch die Neutralisierung mit Säure erzeugt würde; vergleiche dazu die Anmerkungen weiter oben.

Es versteht sich, dass die Gesamtmenge an eingesetzter Base vorteilhafterweise der Gesamtmenge an eingesetzten organischen Basen entspricht, das heißt neben der bzw. den eingesetzten organischen Basen sollten keine weiteren Basen im erfindungsgemäßen Verfahren eingesetzt werden, welche die Umsetzung des Indanons der Formel (II) mit dem Aldehyd der Formel (III) katalysieren. Der Fachmann kann die für die komplette Umsetzung der eingesetzten Edukte erforderliche Menge an organischer Base anhand einfacher Vorversuche bestimmen. Beispielsweise wird der Fachmann dazu bei ansonsten gleichen Reaktionsbedingungen die Basenmenge schrittweise erhöhen, bis keine Erhöhung der Ausbeute mehr erreicht wird.

Vorzugsweise erfolgt das Umsetzen des Indanons der Formel (II) und des aromatischen Aldehyds der Formel (III) ohne Zugabe von einer oder mehreren anorganischen Basen.

In bevorzugten erfindungsgemäßen Verfahren ist der pKₛ-Wert in Wasser der korrespondierenden Säure der verwendeten organischen Base größer als 14,0, bevorzugt größer als 14,5, vorzugsweise größer als 15,0 ist.

Unter einer "Base" wird im Rahmen des vorliegenden Textes ein Stoff, verstanden der Protonen aufnimmt (Protorienakzeptor).

Vorzugsweise umfasst ein erfindungsgemäßes Verfahren den folgenden Schritt bzw. die folgenden Schritte:
Entfernen von während der Umsetzung gebildetem Wasser aus dem Reaktionsgemisch mittels azeotroper Destillation
und/oder
Entfernen von während der Umsetzung gebildetem Wasser aus dem Reaktionsgemisch mittels Adsorption an einen Wasseradsorber, bevorzugt an ein Molekularsieb.

Besonders vorteilhaft ist hierbei die Verfahrensgestaltung, bei der eine azeotrope Destillation durchgeführt wird, denn diese Verfahrensgestaltung ist technisch besonders vorteilhaft. Vorteilhafterweise besitzt das im erfindungsgemäßen Verfahren eingesetzte mit Wasser ein Azeotrop bildende Lösungsmittel eine Löslichkeit in Wasser, die bei 20°C geringer ist als 20 g Lösungsmittel pro Liter Wasser, bevorzugt geringer ist als 5 g Lösungsmittel pro Liter Wasser, besonders bevorzugt geringer ist als 2 g Lösungsmittel pro Liter Wasser.

Im Rahmen des vorliegenden Textes werden Lösungsmittel die bei 20°C eine Löslichkeit in Wasser besitzen, die geringer ist als 20 g Lösungsmittel pro Liter Wasser, als bei 20°C sehr schlecht wasserlösliche Lösungsmittel verstanden.

Vorzugsweise werden neben dem mit Wasser ein Azeotrop bildenden Lösungsmittel geringer Löslichkeit in Wasser im erfindungsgemäßen Verfahren keine weiteren mit Wasser ein Azeotrop bildenden Lösungsmittel eingesetzt, die eine Löslichkeit in Wasser besitzen, die bei 20°C größer ist als 20 g (bevorzugt 5 g, besonders bevorzugt 2 g) Lösungsmittel pro Liter Wasser.

Es versteht sich, dass bei Einsatz der als organische Base bevorzugten oben genannten Verbindungen im Reaktionsgemisch durch Hydrolyse Alkohole entstehen, diese jedoch nicht im vorstehenden Sinne als solche "Lösungsmittel" betrachtet werden, in denen das Indanon der Formel (II) gelöst wird.

In einem erfindungsgemäßen Verfahren kann gemäß einer Alternative das Indanon der Formel (II) in einer ein Lösungsmittel enthaltenden Mischung gelöst werden, wobei das Lösungsmittel ausgewählt ist aus der Gruppe der Lösungsmittel, die mit Wasser ein Azeotrop bilden. Neben dem besagten Lösungsmittel, welches mit Wasser ein Azeotrop bildet, können in der Mischung weitere Lösungsmittelbestandteile vorhanden sein, die - für sich betrachtet - ebenfalls mit Wasser ein Azeotrop bilden bzw. bilden würden. Die Mischung kann jedoch neben einem einzigen Lösungsmittel, welches mit Wasser ein Azeotrop bildet, auch weitere Mischungsbestandteile in gelöster, emulgierter oder suspendierter Form umfassen, welche mit Wasser kein Azeotrop bilden. Beispielsweise kann ein mit Wasser ein Azeotrop bildendes Lösungsmittel wie Toluol in Mischung mit einer oder zwei weiteren organischen Verbindungen eingesetzt werden, die mit Wasser ein Azeotrop bilden oder aber mit Wasser kein Azeotrop bilden.

Wird das Indanon der Formel (II) in einer ein Lösungsmittel enthaltenden Mischung gelöst, wobei das Lösungsmittel ausgewählt ist aus der Gruppe der Lösungsmittel, die mit Wasser ein Azeotrop bilden, so ist es vorteilhaft, wenn die Mischung vollständig oder zu mehr als 50 Gew.-% (bevorzugt mehr als 80 Gew.-%) aus diesem mit Wasser ein Azeotrop bildenden Lösungsmittel besteht.

Besonders bevorzugt sind erfindungsgemäße Verfahren, in denen das mit Wasser ein Azeotrop bildende Lösungsmittel ausgewählt ist aus der Gruppe bestehend aus Benzol, Toluol, Xylol, n-Hexan, n-Heptan, Cyclohexan, Methylcyclohexan und Tetrachlormethan, vorzugsweise aus der Gruppe bestehend aus Toluol und Xylol.

Erfindungsgemäß wird das Indanon der Formel (II) in einem Lösungsmittel oder in einer ein Lösungsmittel enthaltenden Mischung gelöst.

Sofern das Indanon der Formel (II) in einem (einzigen) Lösungsmittel gelöst wird (also nicht in einer Mischung) ist dieses Lösungsmittel vorzugsweise ausgewählt aus der oben genannten Gruppe; bevorzugt ist das Lösungsmittel Toluol oder Xylol.

Sofern das Indanon der Formel (II) in einer ein Lösungsmittel enthaltenden Mischung gelöst wird, ist das oder zumindest einer der Lösungsmittel in der Mischung bevorzugt ausgewählt aus der oben genannten Gruppe; bevorzugt ist das Lösungsmittel Toluol oder Xylol.

Sofern das Indanon der Formel (II) in einer ein Lösungsmittel enthaltenden Mischung gelöst wird, besteht die Mischung vorzugsweise vollständig oder zu mehr als 50 Gew.-% (bevorzugt mehr als 80 Gew.-% aus einem oder mehreren Lösungsmitteln, die ausgewählt sind aus der oben genannten Gruppe; bevorzugt besteht die Mischung vollständig aus Toluol und Xylol oder zu mehr als 50 Gew.-% (bevorzugt mehr als 80 Gew.%) aus Toluol und/oder Xylol.

Der Einsatz eines bei 20°C sehr schlecht wasserlöslichen, aber mit Wasser ein Azeotrop bildenden Lösungsmittels ermöglicht es bei Durchführung einer azeotropen Destillation (siehe dazu oben), das Reaktionswasser sicher aus dem Reaktionsgemisch zu entfernen, das bei der azeotropen Destillation mit abgetrennte Lösungsmittel jedoch (beispielsweise unter Nutzung eines üblichen Wasserabscheiders) zurückzugewinnen und in das Reaktionsgemisch zurückzuführen.

In einem erfindungsgemäßen Verfahren liegt das Stoffmengenverhältnis der eingesetzten Menge des Indanons der Formel (II) zur eingesetzten Menge des Aldehyds der Formel (III) bevorzugt im Bereich von 0,6 bis 1,4, vorzugsweise im Bereich von 0,8 bis 1,0. Es hat sich gezeigt, dass bei einem Stoffmengenverhältnis >1,4 die Reaktionsführung vergleichsweise unökonomisch ist. Das im Überschuss eingesetzte Indanon der Formel (II) muss nach der Reaktion aus dem Produktgemisch entfernt werden. Ein Überschuss von Indanon der Formel (II) ist generell aus ökonomischen Gründen weniger vorteilhaft als ein Überschuss an Aldehyd der Formel (III). Bei einem hohen Gehalt an Indanon der Formel (II) im Produktgemisch steigt der erforderliche Aufwand, um das Produkt in ausreichender Reinheit zu erhalten. Dazu muss das Produkt unter Umständen mehrfach aufgereinigt (z.B. umkristallisiert) werden. Das im Überschuss verwendete und nach dem Aufreinigen zurückgewonnene Indanon der Formel (II) liegt in der Regel nicht in ausreichen reiner Form vor und kann daher ohne weitere Aufbereitung nicht wiederverwendet werden.

Nach erfolgter Umsetzung liegt in einem erfindungsgemäßen Verfahren ein Produktgemisch vor, welches sich aus dem anfänglich vorliegenden Reaktionsgemisch entwickelt hat. Vorteilhafterweise wird in einem erfindungsgemäßen Verfahren das Produktgemisch aufgearbeitet. Vorzugsweise wird zur Aufarbeitung des nach erfolgter Umsetzung vorliegenden Produktgemisches die folgenden Schritte durchgeführt:
- Neutralisieren des Produktgemisches,
- Abdestillieren von Lösungsmittel aus dem neutralisierten Produktgemisch,
- vorzugsweise Destillieren des Produktes,
- anschließend vorzugsweise Umkristallisieren des Produkts.

Auf diese Weise wird ein Produkt mit hoher Reinheit erhalten. Durch das Umkristallisieren des Produktes wird ein besonders hoher Reinheitsgrad erreicht.

Im Interesse einer ökonomisch vorteilhaften Verfahrensgestaltung wird im bevorzugten erfindungsgemäßen Verfahren das Umsetzen des Indanons der Formel (II) mit dem aromatischen Aldehyd der Formel (III) solange durchgeführt, bis ein Umsatz von zumindest >70 %, bevorzugt >80 %, besonders bevorzugt >90% erreicht ist. Der Umsatz wird dabei auf die eingesetzte Menge des im Unterschuss verwendeten Eduktes bezogen. Sofern das Indanon der Formel (II) und das aromatische Aldehyd der Formel (III) äquimolar verwendet werden, wird der Umsatz auf die eingesetzte Menge des Indanons der Formel (II) bezogen.

Der Fachmann kann das Fortschreiten der Reaktion in üblicher Weise mittels Gaschromatographie verfolgen; zur Bestimmung des Umsatzes zu einem bestimmten Zeitpunkt wird der Fachmann eine Probe aus dem Reaktionsgemisch entnehmen und nach entsprechender Kalibrierung in üblicher Weise eine gaschromatographische Untersuchung vornehmen.

Zum Erreichen besonders hervorragender Verfahrensergebnisse, das heißt insbesondere zum Erreichen besonders reiner Produkte werden in bevorzugten erfindungsgemäßen Verfahren folgende Schritte vor dem Umsetzen des Indanons der Formel (II) mit dem aromatischen Aldehyd der Formel (III) durchgeführt:
- Waschen der nach dem Lösen des Indanons der Formel (II) vorliegenden Lösung mit einer wässrigen alkalischen Lösung und Abtrennen der wässrigen Phase nach dem Waschen,
- vorzugsweise Waschen der resultierenden Lösung mit einer Salzlösung, bevorzugt mit einer NaCl-Lösung,
- vorzugsweise Entfernen von nach dem Waschen verbliebenem Restwasser aus der organischen Phase, vorzugsweise mittels azeotroper Destillation.

Diese bevorzugte Ausgestaltung eines erfindungsgemäßen Verfahrens führt zu einem Produkt, welches eine besondere Reinheit besitzt und insbesondere nahezu frei von den oben diskutierten Nebenprodukten ist, welche sich vom angestrebten Zielprodukt lediglich dadurch unterscheiden, dass es keine Doppelbindung am Kohlenstoff in 2-Position des 5-Rings der Formel (II) besitzt. Dies trifft insbesondere für erfindungsgemäße Verfahren zur Herstellung von 2-Benzyliden-3,3-dimethyl-5,6-dimetoxy-1-indanon zu. Üblicherweise erfolgt die Herstellung des in der bevorzugten erfindungsgemäßen Ausgestaltung eingesetzten 3,3-Dimethyl-5,6-dimethoxy-1-indanons aus den Edukten Veratrol (1,2-Dimethoxybenzol) und 3,3-Dimethylacrylsäure in Anwesenheit großer Mengen von Polyphosphorsäure. Trotz gründlicher Aufbereitung sind üblicherweise noch geringe Säuremengen in dem 3,3-Dimethyl-5,6-dimethoxy-1-indanon vorhanden. Bei der bevorzugten erfindungsgemäßen Ausgestaltung, die das Waschen der nach dem Lösen des (wie beschrieben üblicherweise durch Säure verunreinigten) 3,3-Dimethyl-5,6-dimethoxy-1-indanons vorliegenden Lösung in einer wässrigen alkalischen Lösung und das Abtrennen der wässrigen Phase nach dem Waschen umfassen, ist die Bildung des 2-Benzyl-3,3-dimethyl-5,6-dimethoxy-1-indanon (als Nebenprodukt) drastisch reduziert. Zudem resultiert ein Produkt, welches nur noch eine äußerst geringe Gelbfärbung besitzt, vergleiche hierzu die Beispiele weiter unten.

Gemäß dem Vorgesagten umfasst ein besonders bevorzugtes erfindungsgemäßes Verfahren die folgenden Schritte:
- Bereitstellen oder Herstellen eines Indanons der Formel (II) und eines Aldehyds der Formel (III) wie vorstehend definiert,
- Lösen des Indanons in dem Lösungsmittel oder in einer das Lösungsmittel enthaltenden Mischung, so dass eine Lösung des Indanons resultiert,
- Waschen der nach dem Lösen des Indanons der Formel (II) vorliegenden Lösung mit einer wässrigen alkalischen Lösung und Abtrennen der wässrigen Phase nach dem Waschen,
- vorzugsweise Waschen der resultierenden Lösung mit einer Salzlösung, bevorzugt mit einer NaCl-Lösung,
- vorzugsweise Entfernen von nach dem Waschen verbliebenem Restwasser aus der organischen Phase, vorzugsweise mittels azeotroper Destillation,
- Zugeben der organischen Base und vorzugsweise schrittweise des Aldehyds zu der vorzugsweise von Restwasser befreiten Lösung des Indanons, so dass ein Reaktionsgemisch gebildet wird, in dem sich das Indanon der Formel (II) mit dem aromatischen Aldehyd der Formel (III) umsetzt,
- Entfernen von während der Umsetzung gebildetem Wasser aus dem Reaktionsgemisch mittels azeotroper Destillation,
- Neutralisieren des nach erfolgter Umsetzung vorliegenden Produktgemisches,
- Abdestillieren von Lösungsmittel aus dem neutralisierten Produktgemisch,
- vorzugsweise Destillieren des Produktes,
- anschließend vorzugsweise Umkristallisieren des Produkts.

Als Indanon der Formel (II) wird dabei entsprechend dem Vorgesagten vorzugsweise 3,3-Dimethyl-5,6-dimethoxy-1-indanon und als aromatischer Aldehyd der Formel (III) wird vorzugsweise Benzaldehyd eingesetzt.

Im bevorzugten erfindungsgemäßen Verfahren erfolgt bei der Aufarbeitung nach dem Neutralisieren des Produktgemisches und dem Abdestillieren von Lösungsmittel aus dem neutralisierten Produktgemisch vorzugsweise das Umkristallisieren des resultierenden Produkts. Das Umkristallisieren des Produktes wird dabei bevorzugt aus einem Lösungsmittel erfolgen, in dem das Produkt bei 20°C schlechter löslich ist als bei der Siedetemperatur des Lösungsmittels, bevorzugt aus einem Alkanol mit 1 bis 4 Kohlenstoffatomen, besonders bevorzugt aus Methanol, Ethanol, Propanol oder einer Mischung von zwei oder mehr dieser Alkanole. Ganz besonders bevorzugt ist die Umkristallisierung aus Ethanol. Es hat sich gezeigt, dass bei Einsatz der besagten Alkanole (insbesondere Ethanol) besonders gute Umkristallisationsergebnisse erreicht werden können. Es versteht sich, dass vor dem Schritt der Umkristallisation die ggf. aus vorherigen Prozessschritten noch vorhandenen Lösungsmittel möglichst vollständig entfernt werden.

Der Fachmann wird die vorstehend diskutierten Verfahrensparameter anhand üblicher Vorversuche vorzugsweise so optimieren, dass er besonders reine Produkte erhält. Insbesondere wird er dabei die folgenden Verfahrensparameter in der oben dargelegten Weise einstellen bzw. auswählen:
- Das verwendete Lösungsmittel wird bevorzugt so ausgewählt, dass es ein Azeotrop mit Wasser bildet, jedoch bei 20°C sehr schlecht wasserlöslich ist. Somit ist es möglich, das bei der Reaktion gebildete Wasser mittels azeotroper Destillation zu entfernen und das Lösungsmittel zurückzuführen.
- Die bei der Reaktion eingestellte Temperatur wird bevorzugt so ausgewählt, dass das verwendete Lösungsmittel siedet, sodass das bei der Reaktion gebildete Wasser mittels azeotroper Destillation entfernt werden kann.
- Das Verhältnis der Menge des Indanons der Formel (II) zur eingesetzten Menge des Aldehyds der Formel (III) wird bevorzugt so eingestellt, dass nur geringe Mengen der verwendeten Edukte im Produktgemisch verbleiben.
- Die verwendete organische Base wird bevorzugt so ausgewählt, dass sie das Indanon der Formel (II) zur Umsetzung mit dem Aldehyd der Formel (III) in das entsprechende Enolat überführt.
- Das Verhältnis der Menge der verwendeten organischen Base zur eingesetzten Menge des Indanons der Formel (II) wird bevorzugt so eingestellt, dass die für die komplette Umsetzung erforderliche Menge an organischer Base erreicht ist, jedoch gleichzeitig möglichst wenig der organischer Base eingesetzt wird.
- Das verwendete Lösungsmittel für die Umkristallisation wird bevorzugt so ausgewählt, dass alle Bestandteile des Produktgemisches nach dem Abdestillieren des für die Reaktion verwendeten Lösungsmittels in der Siedehitze des Lösungsmittels für die Umkristallisation löslich sind, jedoch beim Abkühlen die Verbindung der Formel (I) möglichst vollständig auskristallisiert, während alle weiteren Bestandteile in Lösung verbleiben.
   Insgesamt gelangt der Fachmann so zu einem besonders bevorzugten erfindungsgemäßen Verfahren zur Herstellung einer Verbindung der Formel (I) oder einer Mischung umfassend eine Verbindung der Formel (I), wobei die Verfahrensbedingungen so eingestellt sind, dass das Stoffmengenverhältnis der
- hergestellten Menge der Verbindung der Formel (I) zur
- gegebenenfalls als Nebenprodukt hergestellten Menge der Verbindung der Formel (IV) 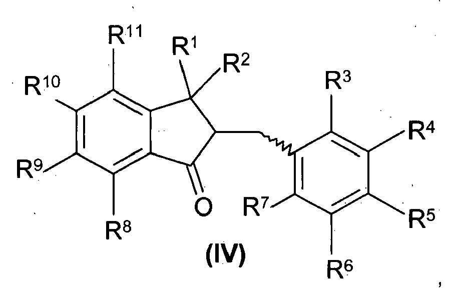 in der die Bedeutung der Gruppen R¹, R², R³, R⁴, R⁵, R⁶, R⁷, R⁸, R⁹, R¹⁰, R¹¹ und R¹² die gleiche ist wie die Bedeutung der gleich bezeichneten Gruppen in der Verbindung der Formel (I),
größer ist als 50, vorzugsweise größer als 100, bevorzugt größer als 1000.

Die vorliegende Erfindung betrifft auch eine Mischung umfassend eine Verbindung der Formel (I) wie hinsichtlich der erfindungsgemäßen Verfahren vorstehend definiert,
- gegebenenfalls eine entsprechende Verbindung der Formel (IV) wie vorstehend definiert (das heißt eine Verbindung der Formel (IV), die sich lediglich durch Abwesenheit einer Doppelbindung am Kohlenstoff in 2-Position des 5-Rings der Formel (II) von der in der Mischung enthaltenen Verbindung der Formel (I) unterscheidet),
   wobei das Stoffmengenverhältnis der Gesamtmenge der Verbindung der Formel (I) zur gegebenenfalls vorliegenden Gesamtmenge der Verbindung der Formel (IV) größer ist als 50, vorzugsweise größer als 100, bevorzugt größer als 1000,
- gegebenenfalls eine die Mischung gelb färbende Substanz,
- eine Menge an Lösungsmittel ausgewählt aus der Gruppe der Lösungsmittel, die mit Wasser ein Azeotrop bilden und bei 20°C sehr schlecht wasserlöslich sind, wobei das mit Wasser ein Azeotrop bildende Lösungsmittel eine Löslichkeit in Wasser besitzt, die bei 20°C geringer ist als 20 g Lösungsmittel pro Liter Wasser,
- sowie gegebenenfalls weitere Bestandteile,
herstellbar nach einem erfindungsgemäßen Verfahren wie vorstehend definiert.

Für die erfindungsgemäße Mischung und die in der Mischung enthaltenen Bestandteile gelten sämtliche vorstehend gemachten Ausführungen im Zusammenhang mit dem erfindungsgemäßen Verfahren. Dies gilt insbesondere für die ausgewählten Lösungsmittel.

Der Fachmann kann die erfindungsgemäße Mischung anhand der Anwesenheit einer Restmenge an Lösungsmittel, die mit Wasser ein Azeotrop bildet, jedoch bei 20°C sehr schlecht wasserlöslich ist, von Mischungen unterscheiden, welche aus dem Stand der Technik bereits bekannt sind.

Zwar ist es für die kosmetische Praxis vorteilhaft, wenn eine Mischung, welche eine Verbindung der Formel (I) umfasst, lediglich sehr geringe oder keine Mengen an dem besagten Lösungsmittel umfasst, doch hat sich in eigenen Untersuchungen gezeigt, dass im erfindungsgemäßen Verfahren auch bei Durchführung einer Umkristallisation noch immer (äußerst geringe) Mengen an Lösungsmittel im Produktgemisch verbleiben. Typische Mengen an Lösungsmittel, die auch nach Umkristallisierung im Produktgemisch verbleiben, sind geringer als 0,01 Gew-%, bei besonders sorgfältiger Aufbereitung in manchen Fällen geringer als 0,001 Gew-%. Üblicherweise ist die verbleibende Menge an Lösungsmittel jedoch größer als 0,0001 Gew-%, so dass das Produktgemisch üblicherweise das Lösungsmittel in einer Menge enthält, die im Bereich von 0,0001 Gew-% bis 0,01 Gew-%, vorzugsweise im Bereich von 0,0001 Gew-% bis 0,001 Gew-% liegt, bezogen auf die Gesamtmasse des Produktgemisches.

In einer bevorzugten erfindungsgemäßen Mischung beträgt der Anteil an der Verbindung der Formel (I) mehr als 99,5 Gew.-%, bevorzugt mehr als 99,9 Gew.%, besonders bevorzugt mehr als 99,99 Gew.-%, bezogen auf die Gesamtmasse der Mischung.

Die vorliegende Erfindung betrifft auch bevorzugte erfindungsgemäße Mischungen, die kosmetische oder pharmazeutische Formulierungen sind. Eine solche erfindungsgemäße Mischung ist vorzugsweise zur Anwendung als Hautschutzmittel, Lichtschutzmittel und/oder AhR-Antagonist vorgesehen.

Die vorliegende Erfindung betrifft auch ein Verfahren zur Herstellung eines Hautschutzmittels, Lichtschutzmittels oder einer Zusammensetzung mit AhRantagonistischer Wirkung, umfassend die folgenden Schritte:
- Herstellen einer Verbindung der Formel (I) nach einem erfindungsgemäßen Verfahren oder Bereitstellen einer erfindungsgemäßen Mischung;
- Vermischen der hergestellten Verbindung bzw. der bereitgestellten Mischung mit kosmetischen bzw. pharmazeutischen Hilfsstoffen.

Die Erfindung wird nachfolgend anhand von Beispielen im Detail erläutert.

### Methode zur Bestimmung von L*a*b-Farbwerten:

Die L*a*b-Farbwerte werden gemäß DIN EN ISO 11664-4:2011-07 bestimmt. Die Messungen wird mit einem Lico-300-Gerät der Firma Hach Lange, Düsseldorf durchgeführt. Nachfolgend werden lediglich ermittelte L-Werte angegeben. Der L-Wert gibt die Helligkeit der Probe wieder. Ein L-Wert von 100 entspricht einer weißen Probe, ein L-Wert von 0 entspricht einer schwarzen Probe.

Vergleichsbeispiel 1 (vgl. WO2007/128723 A1, Beispiel 1): Herstellung von 2-Benzyliden-3,3-dimethyl-5,6-dimethoxy-1-indanon:

### Vergleichsbeispiel 1a. Umkristallisierung aus Methanol:

In 100g Diethylenglykoldimethylether wurden 0,17 mol festes Kaliumhydroxid suspendiert und 0,40 mol 3,3-Dimethyl-5,6-dimethoxy-1-indanon zugesetzt. Die Mischung wurde unter Rühren auf 80°C erhitzt. Innerhalb von einer Stunde wurden 0,61 mol Benzaldehyd hinzudosiert und die Lösung wurde weitere drei Stunden bei 80°C gerührt. Zur Aufarbeitung wurde auf Raumtemperatur abgekühlt, 400g Eiswasser zugegeben und mit 30g 10%igen wässrigen Salzsäurelösung neutral gestellt. Nach Extraktion mit 400g Methyl-*tert*-butylether wurde das Produkt aus Methanol umkristallisiert. Man erhielt 96,2 g (78 Gew.-% d. Theorie bezogen auf das 3,3-Dimethyl-5,6-dimethoxy-1-indanon) eines gelben Feststoffes. Das Produkt wies laut Routine-GC-Untersuchungen eine Reinheit von 99,6 Gew.-% (2-Benzyliden-3,3-dimethyl-5,6-dimethoxy-1-indanon) auf und enthielt 0,3 Gew.-% 2-Benzyl-3,3-dimethyl-5,6-dimethoxy-1-indanon und 0,1% weitere Verunreinigungen. Von dem hergestellten Produkt wurde eine L*a*b-Farbmessung durchgeführt. Der L-Wert des Produktes betrug 88,58.

### Vergleichsbeispiel 1b. Umkristallisierung aus Ethanol:

Bei alternativer Umkristallisierung aus Ethanol wurden nahezu identische Ergebnisse erzielt.

### Vergleichsbeispiel 2: Herstellung von 2-Benzyliden-3,3-dimethyl-5,6-dimethoxy-1-indanon:

### Vergleichsbeispiel 2a. Umkristallisierung aus Methanol:

Die Durchführung in Vergleichsbeispiel 2a entspricht der Durchführung gemäß Vergleichsbeispiel 1a, jedoch wurden statt 100g Diethylenglykoldimethylether 100g Diethylenglykoldiethylether als Lösungsmittel verwendet.

Man erhielt 90,0 g (73 Gew.-% d. Theorie bezogen auf das 3,3-Dimethyl-5,6-dimethoxy-1-indanon) eines gelben Feststoffes. Das Produkt wies laut GC-Untersuchungen eine Reinheit von 99,6 Gew.-% (2-Benzyliden-3,3-dimethyl-5,6-dimethoxy-1-indanon) auf und enthielt 0,3 Gew.-% 2-Benzyl-3,3-dimethyl-5,6-dimethoxy-1-indanon und 0,1 Gew.-% weitere Verunreinigungen.

### Vergleichsbeispiel 2b. Umkristallisierung aus Ethanol:

Bei alternativer Umkristallisierung aus Ethanol wurden nahezu identische Ergebnisse erzielt.

### Vergleichsbeispiel 3: Herstellung von 2-Benzyliden-3,3-dimethyl-5,6-dimethoxy-1-indanon:

### Vergleichsbeispiel 3a. Umkristallisierung aus Methanol:

Die Durchführung in Vergleichsbeispiel 3a entspricht der Durchführung gemäß Vergleichsbeispiel 2a, jedoch wurden 10 mmol Kaliumhydroxid verwendet. Es wurden nur 2g einer 10%igen wässrigen Salzsäurelösung eingesetzt, um die Lösung neutral zu stellen. Man erhielt 123g eines gelben Edukt-Produkt-Gemisches. Mittels GC-Messungen konnte ermittelt werden, dass das gelbe Edukt-ProduktGemisch 19,7g (16 Gew.-% d. Theorie bezogen auf das 3,3-Dimethyl-5,6-dimethoxy-1-indanon) des Produktes enthielt.

### Vergleichsbeispiel 3b. Umkristallisierung aus Ethanol:

Bei alternativer Umkristallisierung aus Ethanol wurden nahezu identische Ergebnisse erzielt.

### Vergleichsbeispiel 4: Herstellung von 2-Benzyliden-3,3-dimethyl-5,6-dimethoxy-1-indanon:

### Vergleichsbeispiel 4a. Umkristallisierung aus Methanol:

Die Durchführung in Vergleichsbeispiel 4a entspricht der Durchführung gemäß Vergleichsbeispiel 2a, jedoch wurden 0,7g (12 mmol) Kaliumhydroxid verwendet. Als Lösungsmittel wurden statt 100g Diethylenglykoldiethylether 100 g Toluol verwendet. Es wurden nur 2g einer 10%igen wässrigen Salzsäurelösung eingesetzt, um die Lösung neutral zu stellen. Man erhielt 21,0 g (17 Gew.-% d. Theorie bezogen auf das 3,3-Dimethyl-5,6-dimethoxy-1-indanon) eines gelben Feststoffes.

### Vergleichsbeispiel 4b. Umkristallisierung aus Ethanol:

Bei alternativer Umkristallisierung aus Ethanol wurden nahezu identische Ergebnisse erzielt.

### Beispiel 1: Herstellung von 2-Benzyliden-3,3-dimethyl-5,6-dimethoxy-1-indanon:

### Beispiel 1a. Umkristallisierung aus Methanol:

In 180g Toluol wurden 89g (0,40 mol) 3,3-Dimethyl-5,6-dimethoxy-1-indanon gelöst, das auf übliche Weise hergestellt wurde. Diese Lösung wurde mit 60mL einer 5%igen wässrigen Lösung von Kaliumhydroxid und dann zweimal mit jeweils 50mL einer 10%igen wässrigen Kochsalzlösung gewaschen. Anschließend wurde die organische Lösung an einem Wasserabscheider azeotrop vom Restwasser befreit. Im Wasserabscheider fielen ca. 80g Toluol (obere Phase) sowie ca. 2g Wasser (untere Phase) an.

Zu der trockenen Lösung des 3,3-Dimethyl-5,6-dimethoxy-1-indanon in Toluol wurden 1,1g (10 mmol) Kalium-*tert*-butanolat gegeben; die resultierende Mischung wurde unter Rückfluss (ca. 110°C) gerührt. Innerhalb von 1h wurden 65g (0,61 mol) Benzaldehyd hinzudosiert und die Lösung wurde weitere 3 h bei besagter Temperatur gerührt. Während der gesamten Reaktionsdauer wurde das entstehende Reaktionswasser mittels eines Wasserabscheiders azeotrop abdestilliert. Zur Aufarbeitung wurde auf Raumtemperatur abgekühlt, 400g Eiswasser wurden zugegeben und die Lösung wurde mit 3g einer 10%igen wässrigen Salzsäurelösung neutral gestellt. Die verbleibende organische Phase wurde mit 100g einer 10%igen wässrigen Kochsalzlösung gewaschen, das verbleibende Lösungsmittel wurde abdestilliert. Anschließend wurde das Rohprodukt mittels einer Kurzweg-Destillationsanlage bei einem Druck von ca. 1mbar und einer Temperatur von ca. 170 °C abdestilliert. Nach Kristallisation des Destillats aus Methanol wurden 82g (66% d. Theorie bezogen auf das 3,3-Dimethyl-5,6-dimethoxy-1-indanon) des 2-Benzyliden-3,3-dimethyl-5,6-dimethoxy-1-indanons als hellgelben Feststoff erhalten. Die Reinheit des Produktes betrug >99,9 Gew.-%. Das bei den Vergleichsbeispielen beobachtete Nebenprodukt 2-Benzyl-3,3-dimethyl-5,6-dimethoxy-1-indanon war mittels Routine-GC-Untersuchungen nicht detektierbar. Von dem hergestellten Produkt wurde eine L*a*b-Farbmessung durchgeführt. Der L-Wert des Produktes betrug 94,09.

### Beispiel 1b. Umkristallisierung aus Ethanol:

Bei alternativer Umkristallisierung aus Ethanol wurden nahezu identische Ergebnisse erzielt.

### Beispiel 2: Herstellung von 2-Benzyliden-3,3-dimethyl-5,6-dimethoxy-1-indanon:

### Beispiel 2a. Umkristallisierung aus Methanol:

Die Durchführung in Beispiel 2a entspricht der Durchführung gemäß Beispiel 1a, jedoch wurde auf eine Destillation des Rohproduktes verzichtet. Nach Kristallisation des Rohproduktes aus Methanol wurden 86g (69% d. Theorie bezogen auf das 3,3-Dimethyl-5,6-dimethoxy-1-indanon) des 2-Benzyliden-3,3-dimethyl-5,6-dimethoxy-1-indanons als hellgelben Feststoff erhalten. Die Reinheit des Produktes betrug >99,9 Gew.-%. Das bei den Vergleichsbeispielen beobachtete Nebenprodukt 2-Benzyl-3,3-dimethyl-5,6-dimethoxy-1-indanon (Verbindung der Formel (V)) war mittels Routine-GC-Untersuchungen nicht detektierbar. Von dem hergestellten Produkt wurde eine L*a*b-Farbmessung durchgeführt. Der L-Wert des Produktes betrug 90,15.

### Beispiel 2b. Umkristallisierung aus Ethanol:

Bei alternativer Umkristallisierung aus Ethanol wurden nahezu identische Ergebnisse erzielt.

### Beispiel 3: Prüfung der Kristallisation in Emulsion:

Entsprechend den Angaben in der nachfolgenden Tabelle 1 wurden zwei kosmetische Formulierungen als Emulsionen [E(I) und E(II)] hergestellt. Das Produkt aus Beispiel 1a enthielt reines 2-Benzyliden-3,3-dimethyl-5,6-dimethoxy-1-indanon und wurde in der Emulsion E(I) verwendet. Für die Emulsion E(II) wurde ebenfalls 2-Benzyliden-3,3-dimethyl-5,6-dimethoxy-1-indanon aus Beispiel 1a eingesetzt, aber es wurde so mit Benzyl-3,3-dimethyl-5,6-dimethoxy-1-indanon verunreinigt, dass eine 5 Gew.-%ige Verunreinigung vorlag, bezogen auf die Gesamtmasse des verunreinigten Produktes.

Die Emulsionen wurden abgedeckt und bei Raumtemperatur für 3 Tage stehen gelassen. Anschließend wurden die Emulsionen unter einem Mikroskop betrachtet. Emulsion E(I) wies keine Kristalle auf. In Emulsion E(II) konnten Kristallkeime mit einer Größe von 4 bis 7µm beobachtet werden.

Dies bedeutet, dass bereits ein Anteil von 250 ppm (0,025 Gew.-%) des Nebenproduktes (Benzyl-3,3-dimethyl-5,6-dimethoxy-1-indanon) in der kosmetischen Formulierung die Bildung von Kristallisationskeimen initiiert, während eine nebenproduktfreie Formulierung keine Kristallisation aufweist.

**Tabelle 1: Rezeptur der für die Kristallisationsversuche verwendeten kosmetischen Formulierung:**

| **Phase** | **Rohstoff** | **INCI** | **E(I) Gew.-%** | **E(II) Gew.-%** |
|---|---|---|---|---|
| **A** | Wasser dem | Aqua | 84,15 | 84,15 |
| | Symdiol 68 | 1,2-Hexanediol, Gaprylyl Glycol | 0,60 | 0,60 |
| | SymMoillent W/S | Trideceth-9, PEG-5 Isononanoate, Aqua | 0,50 | 0,50 |
| | Glycerin 85% | Glycerin | 1,00 | 1,00 |
| | Hydrolite 5 | 1,2-Pentylene Glycol | 1,00 | 1,00 |
| **B** | PCL Liquid 100 | Cetearyl Octanoate | 3,00 | 3,00 |
| | Lanette O | Cetearyl Alkohol | 2,00 | 2,00 |
| | Produkt aus Beispiel 1a | Benzylidene Dimethoxydimethylindanone | 0,50 | --- |
| | Produkt aus Beispiel 1a versetzt mit 5 Gew.-% Benzyl-3,3-dimethyl- 5,6-dimethoxy-1-indanon | Benzylidene Dimethoxydimethylindanone; | ---- | 0,50 |
| | Pemulen TR1 | Acylates/C10-30Alkyl Acrylate Crosspolymer | 0,20 | 0,20 |
| | Carbopol Ultrez-21 | Acylates/C10-30Alkyl Acrylate Crosspolymer | 0,05 | 0,05 |
| | Paraffinöl 5° | MineralOil | 3,00 | 3,00 |
| | Dragoxat 89 | Ethylhexyl Isononanoate | 3,00 | 3,00 |
| | Abil 350 | Dimethicone | 0,50 | 0,50 |
| **C** | NaOH, 10%aq. | Sodium Hydroxide | 0,50 | 0,50 |
| | **Summe** | | **100,00** | **100,00** |

## Patentansprüche

1. Verfahren zur Herstellung einer Verbindung der Formel (I) 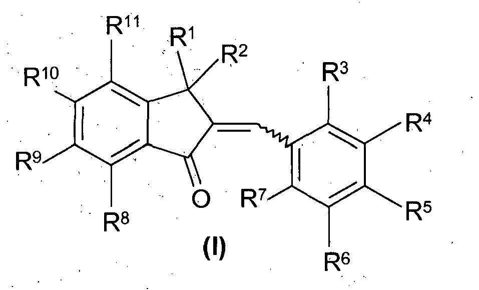 oder einer Mischung umfassend eine Verbindung der Formel (I),
mit folgenden Schritten:
- Lösen eines Indanons der Formel (II) in einem Lösungsmittel oder in einer ein Lösungsmittel enthaltenden Mischung, wobei das Lösungsmittel ausgewählt ist aus der Gruppe der Lösungsmittel, die mit Wasser ein Azeotrop bilden,
- Umsetzen des Indanons der Formel (II) mit einem aromatischen Aldehyd der Formel (III) in Gegenwart zumindest einer organischen Base, gemäß dem Schema 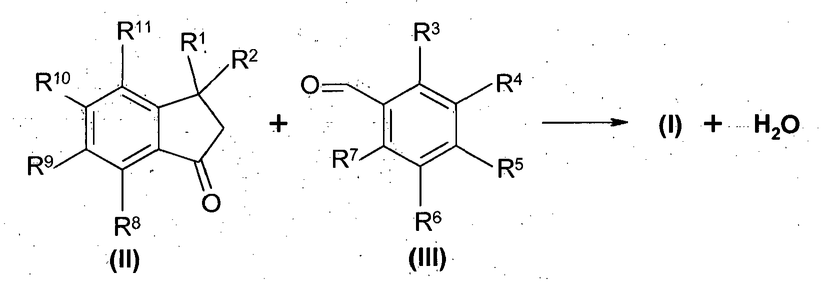 wobei R¹ und R² unabhängig voneinander jeweils Wasserstoff oder eine verzweigte oder unverzweigte Alkylgruppe mit 1 bis 12 Kohlenstoffatomen bedeuten und
wobei R³, R⁴, R⁵, R⁶, R⁷, R⁸, R⁹, R¹⁰ und R¹¹ unabhängig voneinander jeweils Wasserstoff, Hydroxy oder eine verzweigte oder unverzweigte Alkyl- oder Alkoxygruppe mit 1 bis 12 Kohlenstoffatomen bedeuten und
- Entfernen von gebildetem Wasser aus dem Reaktionsgemisch während der Umsetzung.

2. Verfahren nach Anspruch 1, wobei
- R¹ und R² unabhängig voneinander jeweils Methyl, Ethyl, n-Propyl oder iso-Propyl bedeuten und R³, R⁴, R⁵, R⁶, R⁷, R⁸, R⁹, R¹⁰ und R¹¹ unabhängig voneinander jeweils Wasserstoff, Methyl, Ethyl, n-Propyl, iso-Propyl, Methoxy, Ethoxy, Propoxy oder Butoxy bedeuten,
- wobei vorzugsweise R¹ und R² jeweils Methyl bedeuten, R³, R⁶, R⁷, R⁸ und R¹¹ jeweils Wasserstoff bedeuten und R⁴, R⁵, R⁹ und R¹⁰ unabhängig voneinander jeweils Wasserstoff, Methoxy oder n-Propoxy bedeuten,
- besonders bevorzugt R¹ und R² jeweils Methyl bedeuten, R⁹ und R¹⁰ jeweils Methoxy bedeuten und R³, R⁴, R⁵, R⁶, R⁷, R⁸ und R¹¹ jeweils Wasserstoff bedeuten.

3. Verfahren nach einem der vorangehenden Ansprüche, wobei die organische Base ein Alkoholat ist, vorzugsweise ein Alkalimetall- oder Erdalkalimetallalkoholat, vorzugsweise ein Akalimetall- oder Erdalkalimetallalkoholat mit verzweigtem Alkoholatanion, wobei die organische Base vorzugweise ausgewählt ist aus der Gruppe bestehend aus Kalium-*tert*-butanolat, Natrium-*tert*-butanolat, Lithium-*tert-*butanolat, Barium-*tert*-butanolat, Magnesium-*tert*-butanolat, Kalium-*tert*-pentanolat, Natrium-*tert*-pentanolat und Lithium-*tert*-pentanolat, wobei die organische Base bevorzugt ausgewählt ist aus der Gruppe bestehend aus Natrium-*tert*-butanolat und Kalium-*tert*-butanolat.

4. Verfahren nach einem der vorangehenden Ansprüche, wobei das Stoffmengenverhältnis der eingesetzten Menge von organischer Base zur eingesetzten Menge von Indanon der Formel (II) kleiner als 0,50, vorzugsweise kleiner als 0,20, bevorzugt kleiner als 0,10, besonders bevorzugt kleiner 0,05 ist.

5. Verfahren nach einem der vorangehenden Ansprüche, wobei das Stoffmengenverhältnis der eingesetzten Menge des Indanons der Formel (II) zur eingesetzten Menge des Aldehyds derFormel (III) im Bereich von 0,6 bis 1,4 liegt, vorzugsweise im Bereich von 0,8 bis 1,0.

6. Verfahren nach einem der vorangehenden Ansprüche, mit folgendem Schritt bzw. Schritten:
Entfernen von während der Umsetzung gebildetem Wasser aus dem Reaktionsgemisch mittels azeotroper Destillation
und/oder
Entfernen von während der Umsetzung gebildetem Wasser aus dem Reaktionsgemisch mittels Adsorption an einen Wasseradsorber, bevorzugt an ein Molekularsieb.

7. Verfahren nach einem der vorangehenden Ansprüche, wobei das mit Wasser ein Azeotrop bildende Lösungsmittel eine Löslichkeit in Wasser besitzt, die bei 20°C geringer ist als 20 g Lösungsmittel pro Liter Wasser, bevorzugt geringer ist als 5 g Lösungsmittel pro Liter Wasser und besonders bevorzugt geringer ist als 2 g Lösungsmittel pro Liter Wasser.

8. Verfahren nach einem der vorangehenden Ansprüche, wobei das mit Wasser ein Azeotrop bildende Lösungsmittel ausgewählt ist aus der Gruppe bestehend aus Benzol, Toluol, Xylol, n-Hexan, n-Heptan, Cyclohexan, Methylcyclohexan und Tetrachlormethan, vorzugsweise aus der Gruppe bestehend aus Toluol und Xylol.

9. Verfahren nach einem der vorangehenden Ansprüche, wobei zur Aufbereitung des nach erfolgter Umsetzung vorliegenden Produktgemisches die folgenden Schritte durchgeführt werden:
- Neutralisieren des Produktgemisches,
- Abdestillieren von Lösungsmittel aus dem neutralisierten Produktgemisch,
- vorzugsweise Destillieren des Produktes,
- anschließend vorzugsweise Umkristallisieren des Produkts.

10. Verfahren nach einem der vorangehenden Ansprüche, mit folgenden Schritten vor dem Umsetzen des Indanons der Formel (II) mit dem aromatischen Aldehyd der Formel (III):
- Waschen der nach dem Lösen des Indanons der Formel (II) vorliegenden Lösung mit einer wässrigen alkalischen Lösung und Abtrennen der wässrigen Phase nach dem Waschen,
- vorzugsweise Waschen der resultierenden Lösung mit einer Salzlösung, bevorzugt mit einer NaCl-Lösung,
- vorzugsweise Entfernen von nach dem Waschen verbliebenem Restwasser aus der organischen Phase, vorzugsweise mittels azeotroper Destillation.

11. Verfahren nach einem der vorangehenden Ansprüche, mit folgenden Schritten:
- Bereitstellen oder Herstellen eines Indanons der Formel (II) und eines Aldehyds der Formel (III) wie in einem der vorangehenden Ansprüche definiert,
- Lösen des Indanons in dem Lösungsmittel oder in einer das Lösungsmittel enthaltenden Mischung, so dass eine Lösung des Indanons resultiert,
- Waschen der nach dem Lösen des Indanons der Formel (II) vorliegenden Lösung mit einer wässrigen alkalischen Lösung und Abtrennen der wässrigen Phase nach dem Waschen,
- vorzugsweise Waschen der resultierenden Lösung mit einer Salzlösung, bevorzugt mit einer NaCl-Lösung,
- vorzugsweise Entfernen von nach dem Waschen verbliebenem Restwasser aus der organischen Phase, vorzugsweise mittels azeotroper Destillation,
- Zugeben der organischen Base und vorzugsweise schrittweise des Aldehyds zu der vorzugsweise von Restwasser befreiten Lösung des Indanons, so dass ein Reaktionsgemisch gebildet wird, in dem sich das Indanon der Formel (II) mit dem aromatischen Aldehyd der Formel (III) umsetzt,
- Entfernen von während der Umsetzung gebildetem Wasser aus dem Reaktionsgemisch mittels azeotroper Destillation,
- Neutralisieren des nach erfolgter Umsetzung vorliegenden Produktgemisches,
- Abdestillieren von Lösungsmittel aus dem neutralisierten Produktgemisch,
- vorzugsweise Destillieren des Produktes,
- anschließend vorzugsweise Umkristallisieren des Produkts.

12. Verfahren nach einem der vorangehenden Ansprüche, zur Herstellung einer Verbindung der Formel (I) oder einer Mischung umfassend eine Verbindung der Formel (I),
wobei die Verfahrensbedingungen so eingestellt sind, dass das Stoffmengenverhältnis der
- hergestellten Menge der Verbindung der Formel (I) zur
- gegebenenfalls als Nebenprodukt hergestellten Menge der Verbindung der Formel (IV) 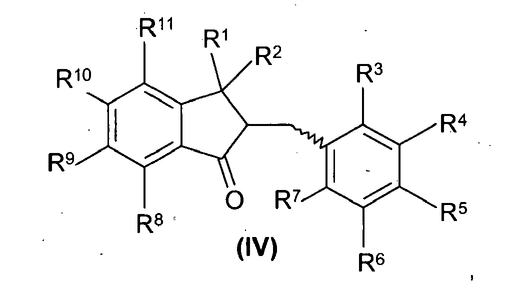 in der die Bedeutung der Gruppen R¹, R², R³, R⁴, R⁵, R⁶, R⁷, R⁸, R⁹, R¹⁰, R¹¹ und R¹² die gleiche ist wie die Bedeutung der gleich bezeichneten Gruppen in der Verbindung der Formel (I),
größer ist als 50, vorzugsweise größer als 100, bevorzugt größer als 1000.

13. Mischung umfassend
- eine Verbindung der Formel (I) wie in einem der vorangehenden Ansprüche definiert,
- gegebenenfalls eine entsprechende Verbindung der Formel (IV) wie in Anspruch 12 definiert,
wobei das Stoffmengenverhältnis der Gesamtmenge der Verbindung der Formel (I) zur gegebenenfalls vorliegenden Gesamtmenge der Verbindung der Formel (IV) größer ist als 50, vorzugsweise größer als 100, bevorzugt größer als 1000,
- gegebenenfalls eine die Mischung gelb färbende Substanz,
- eine Menge an Lösungsmittel ausgewählt aus der Gruppe der Lösungsmittel, die mit Wasser ein Azeotrop bilden und bei 20°C sehr schlecht wasserlöslich sind, wobei das mit Wasser ein Azeotrop bildende Lösungsmittel eine Löslichkeit in Wasser besitzt, die bei 20°C geringer ist als 20 g Lösungsmittel pro Liter Wasser,
- sowie gegebenenfalls weitere Bestandteile,
herstellbar nach einem Verfahren gemäß einem der vorangehenden Ansprüche.

14. Mischung nach Anspruch 13, wobei die Mischung eine kosmetische oder pharmazeutische Formulierung ist.

15. Mischung nach Anspruch 13 oder 14 zur Anwendung als Hautschutzmittel, Lichtschutzmittel und/oder AhR-Antagonist.

## Claims

1. Method for the manufacture of a compound of formula (I) 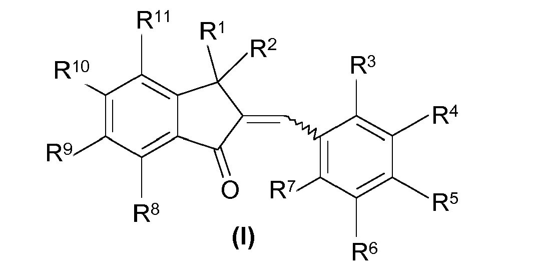 or of a mixture comprising a compound of formula (I),
with the following steps:
- dissolving an indanone of formula (II) in a solvent or in a mixture containing a solvent, wherein the solvent is selected from the group of solvents that form an azeotrope with water,
- conversion of the indanone of formula (II) with an aromatic aldehyde of formula (III) in the presence of at least one organic base, according to the scheme 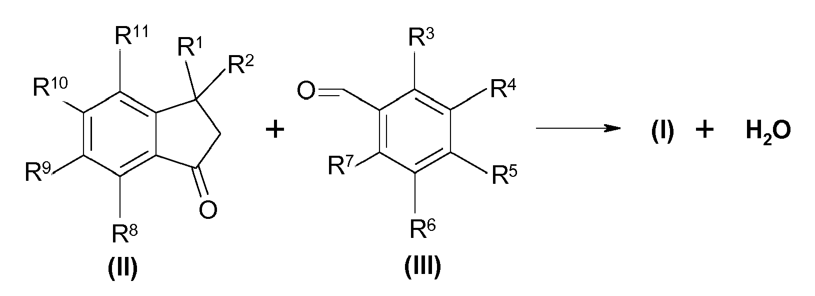 wherein R¹ and R² represent, independently of each other, hydrogen or a branched or unbranched alkyl group with 1 to 12 carbon atoms, respectively, and
wherein R³, R⁴, R⁵, R⁶, R⁷, R⁸, R⁹, R¹⁰ and R¹¹ represent, independently of each other, hydrogen, hydroxy or a branched or unbranched alkyl or alkoxy group with 1 to 12 carbon atoms, respectively, and
- removal of the generated water from the reaction mixture during the conversion.

2. Method according to claim 1, wherein
- R¹ and R² represent, independently of each other, methyl, ethyl, n-propyl or iso-propyl, respectively, and R³, R⁴, R⁵, R⁶, R⁷, R⁸, R⁹, R¹⁰ and R¹¹ represent, independently of each other, hydrogen, methyl, ethyl, n-propyl, iso-propyl, methoxy, ethoxy, propoxy or butoxy, respectively,
- wherein preferably R¹ and R² represent methyl, respectively, R³, R⁶, R⁷, R⁸ and R¹¹ represent hydrogen, respectively, and R⁴, R⁵, R⁹ and R¹⁰ represent, independently of each other, hydrogen, methoxy or n-propoxy, respectively,
- particularly preferably R¹ and R² represent methyl, respectively, R⁹ and R¹⁰ represent methoxy, respectively, and R³, R⁴, R⁵, R⁶, R⁷, R⁸ and R¹¹ represent hydrogen, respectively.

3. Method according to any of the preceding claims, wherein the organic base is an alcoholate, preferably an alkali metal or alkaline earth metal alcoholate, preferably an alkali metal or alkaline earth metal alcoholate with a branched alcoholate anion, wherein the organic base is preferably selected from the group consisting of potassium *tert*-butylate, sodium *tert*-butylate, lithium *tert-*butylate, barium *tert*-butylate, magnesium *tert*-butylate, potassium *tert-*pentylate, sodium *tert*-pentylate and lithium *tert*-pentylate, wherein the organic base is preferably selected from the group consisting of sodium *tert*-butylate and potassium *tert*-butylate.

4. Method according to any of the preceding claims, wherein the molar ratio of the used amount of organic base to the used amount of indanone of formula (II) is smaller than 0.50, preferably smaller than 0.20, preferably smaller than 0.10, particularly preferably smaller than 0.05.

5. Method according to any of the preceding claims, wherein the molar ratio of the used amount of indanone of formula (II) to the used amount of aldehyde of formula (III) is in the range of 0.6 to 1.4, preferably in the range of 0.8 to 1.0.

6. Method according to any of the preceding claims with the following step(s):
Removal of the water that is generated during the conversion from the reaction mixture by means of azeotropic destillation
and/or
Removal of the water that is generated during the conversion from the reaction mixture by means of adsorption to a water adsorber, preferably to a molecular sieve.

7. Method according to any of the preceding claims, wherein the solvent that forms an azeotrope with water has a solubility that is less than 20 g of solvent per litre of water at 20°C, preferably less than 5 g of solvent per litre of water and particularly preferably less than 2 g of solvent per litre of water.

8. Method according to any of the preceding claims, wherein the solvent that forms an azeotrope with water is selected from the group consisting of benzene, toluene, xylene, n-hexane, n-heptane, cyclohexane, methylcyclohexane and carbon tetrachloride, preferably from the group consisting of toluene and xylene.

9. Method according to any of the preceding claims, wherein for treatment of the existing product mixture once conversion has taken place the following steps are performed:
- neutralisation of the product mixture,
- removal of the solvent by distillation from the neutralised product mixture,
- preferably distillation of the product,
- subsequently, preferably recrystallisation of the product.

10. Method according to any of the preceding claims with the following steps before the conversion of the indanone of formula (II) with the aromatic aldehyde of formula (III):
- washing of the solution that is present after dissolving of the indanone of formula (II) with an aqueous, alkaline solution and separation of the aqueous phase after washing,
- preferably washing of the resulting solution with a salt solution, preferably with a NaCl solution,
- preferably removal of residual water, that has remained after washing, from the organic phase, preferably by means of azeotropic destillation.

11. Method according to any of the preceding claims with the following steps:
- provision or production of an indanone of formula (II) and of an aldehyde of formula (III) as defined in any of the preceding claims,
- dissolving of the indanone in the solvent or in a mixture containing the solvent so as to achieve a solution of the indanone,
- washing of the solution that is present after dissolving of the indanone of formula (II) with an aqueous, alkaline solution and separation of the aqueous phase after washing,
- preferably washing of the resulting solution with a salt solution, preferably with a NaCl solution,
- preferably removal of residual water, that has remained after washing, from the organic phase, preferably by means of azeotropic distillation,
- addition of the organic base and, preferably stepwise, of the aldehyde to the solution of the indanone, which preferably is freed from any residual water, so that a reaction mixture is formed in which the indanone of formula (II) reacts with the aromatic aldehyde of formula (III),
- removal of the water, that is generated during the conversion, from the reaction mixture by means of azeotropic distillation,
- neutralisation of the product mixture that is present once the conversion has taken place,
- removal of the solvent by distillation from the neutralised product mixture,
- preferably distillation of the product,
- subsequently, preferably recrystallisation of the product.

12. Method according to any of the preceding claims for manufacture of a compound of formula (I) or a mixture comprising a compound of formula (I),
wherein the process conditions are adjusted such that the molar ratio of the
- produced amount of the compound of formula (I) to
- the amount of the compound of formula (IV) that has been produced as side-product, where appropriate, 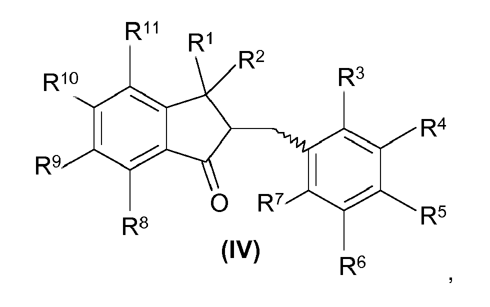 in which the denotation of the groups R¹, R², R³, R⁴, R⁵, R⁶, R⁷, R⁸, R⁹, R¹⁰, R¹¹ and R¹² is the same as the denotation of the groups termed equally in the compound of formula (I),
is larger than 50, preferably larger than 100, preferably larger than 1000.

13. Mixture comprising
- a compound of formula (I) as defined in any one of the preceding claims,
- a corresponding compound of formula (IV) as defined in claim 12, where appropriate,
wherein the molar ratio of the total amount of the compound of formula (I) to the total amount of the compound of formula (IV) that is present where appropriate is larger than 50, preferably larger than 100, preferably larger than 1000,
- a substance that dyes the mixture yellow, where appropriate,
- an amount of solvent selected from the group of solvents that form an azeotrope with water and are very poorly water soluble at 20°C, wherein the solvent that forms an azeotrope with water has a solubility in water that is less than 20 g of solvent per litre of water at 20°C,
- as well as further components, where appropriate,
producible according to a method according to any of the preceding claims.

14. Mixture according to claim 13, wherein the mixture is a cosmetic or pharmaceutical formulation.

15. Mixture according to claim 13 or 14 for use as skin protection agent, light stabiliser and/or AhR antagonist.

## Revendications

1. Procédé de préparation d'un composé de formule (I) 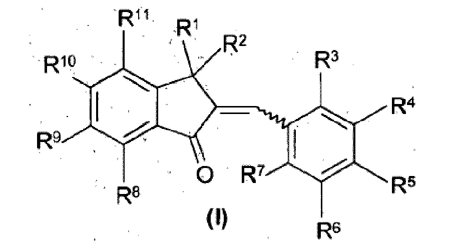 ou d'un mélange comprenant un composé de formule (I),
comprenant les étapes suivantes:
- dissoudre une indanone de formule (II) dans un solvant ou dans un mélange contenant un solvant, le solvant étant choisi dans le groupe des solvants qui forment avec l'eau un azéotrope,
- faire réagir l'indanone de formule (II) avec un aldéhyde aromatique de formule (III), en présence d'au moins une base organique, selon le schéma 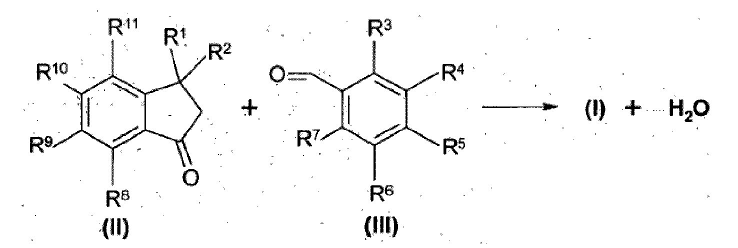 dans lequel R¹ et R² signifient respectivement, indépendamment l'un de l'autre, de l'hydrogène ou un groupe alkyle ramifié ou non ramifié ayant 1 à 12 atomes de carbone, et
dans lequel R³, R⁴, R⁵, R⁶, R⁷, R⁸, R⁹, R¹⁰ et R¹¹ signifient respectivement, indépendamment les uns des autres, de l'hydrogène, un groupe hydroxy ou un groupe alkyle ou alcoxy ramifié ou non ramifié ayant 1 à 12 atomes de carbone, et
- éliminer de l'eau formée, dudit mélange de réaction pendant la réaction.

2. Procédé selon la revendication 1, dans lequel
- R¹ et R² signifient respectivement, indépendamment l'un de l'autre, un groupe méthyle, éthyle, n-propyle ou isopropyle et R³, R⁴, R⁵, R⁶, R⁷, R⁸, R⁹, R¹⁰ et R¹¹ signifient respectivement, indépendamment les uns des autres, de l'hydrogène, un groupe méthyle, éthyle, n-propyle, isopropyle, méthoxy, éthoxy, propoxy ou butoxy,
- de préférence, R¹ et R² signifiant respectivement un groupe méthyle, R³, R⁶, R⁷, R⁸ et R¹¹ signifiant respectivement de l'hydrogène et R⁴, R⁵, R⁹ et R¹⁰ signifiant respectivement, indépendamment les uns des autres, de l'hydrogène, un groupe méthoxy ou n-propoxy,
- de manière particulièrement préférée, R¹ et R² signifient respectivement un groupe méthyle, R⁹ et R¹⁰ signifient respectivement un groupe méthoxy et R³, R⁴, R⁵, R⁶, R⁷, R⁸ et R¹¹ signifient respectivement de l'hydrogène.

3. Procédé selon l'une quelconque des revendications précédentes, dans lequel la base organique est un alcoolate, de préférence un alcoolate de métal alcalin ou de métaux alcalino-terreux, de préférence un alcoolate de métal alcalin ou de métaux alcalino-terreux avec un anion d'alcoolate ramifié, dans lequel la base organique est choisie de préférence dans le groupe constitué par le *tert-*butanolate de potassium, *tert*-butanolate de sodium, le *tert*-butanolate de lithium, le *tert*-butanolate de baryum, le *tert*-butanolate de magnésium, le *tert-*pentanolate de potassium, le *tert*-pentanolate de sodium et le *tert*-pentanolate de lithium, dans lequel la base organique est choisie de préférence dans le groupe constitué par le *tert*-butanolate de sodium et le *tert*-butanolate de potassium.

4. Procédé selon l'une quelconque des revendications précédentes, dans lequel le rapport molaire de la quantité utilisée de base organique à la quantité utilisée d'indanone de formule (II) est inférieur à 0,50, de préférence inférieur à 0,20, de préférence inférieur à 0,10, de manière particulièrement préférée inférieur à 0,05.

5. Procédé selon l'une quelconque des revendications précédentes, dans lequel le rapport molaire de la quantité utilisée de l'indanone de formule (II) à la quantité utilisée de l'aldéhyde de formule (III) est compris entre 0,6 et 1,4, de préférence compris entre 0,8 et 1,0.

6. Procédé selon l'une quelconque des revendications précédentes, comprenant l'étape ou bien les étapes suivantes:
éliminer l'eau formée durant la réaction, dudit mélange de réaction par distillation azéotropique
et/ou
éliminer l'eau formée durant la réaction, dudit mélange de réaction par adsorption sur un adsorbant d'eau, de préférence sur un tamis moléculaire.

7. Procédé selon l'une quelconque des revendications précédentes, dans lequel le solvant formant avec l'eau un azéotrope possède une solubilité dans l'eau qui, à 20 °C, est inférieure à 20 g de solvant par litre d'eau, de préférence inférieure à 5 g de solvant par litre d'eau et, de manière particulièrement préférée, inférieure à 2 g de solvant par litre d'eau.

8. Procédé selon l'une quelconque des revendications précédentes, dans lequel le solvant formant avec l'eau un azéotrope est choisi dans le groupe constitué par le benzène, le toluène, le xylène, le n-hexane, le n-heptane, le cyclohexane, le méthylcyclohexane et le tétrachlorométhane, de préférence dans le groupe constitué par le toluène et le xylène.

9. Procédé selon l'une quelconque des revendications précédentes, dans lequel, pour la préparation du mélange de produit présent après que la réaction s'est produite, les étapes suivantes sont mises en oeuvre:
- neutraliser le mélange de produit,
- distiller du solvant, dudit mélange de produit neutralisé,
- de préférence distiller le produit,
- ensuite, de préférence, recristalliser le produit.

10. Procédé selon l'une quelconque des revendications précédentes, comprenant les étapes suivantes avant la réaction de l'indanone de formule (II) avec l'aldéhyde aromatique de formule (III):
- laver la solution présente après la dissolution de l'indanone de formule (II), avec une solution alcaline aqueuse, et séparer la phase aqueuse après le lavage,
- de préférence, laver la solution résultante avec une solution de sel, de préférence avec une solution de NaCl,
- de préférence, éliminer, de la phase organique, l'eau résiduelle qui est restée après le lavage, de préférence par distillation azéotropique.

11. Procédé selon l'une quelconque des revendications précédentes, comprenant les étapes suivantes:
- fournir ou produire une indanone de formule (II) et un aldéhyde de formule (III), comme défini dans l'une quelconque des revendications précédentes,
- dissoudre l'indanone dans le solvant ou dans un mélange contenant le solvant de sorte qu'il résulte une solution de l'indanone,
- laver la solution présente après la dissolution de l'indanone de formule (II), avec une solution alcaline aqueuse, et séparer la phase aqueuse après le lavage,
- de préférence, laver la solution résultante avec une solution de sel, de préférence avec une solution de NaCl,
- de préférence, éliminer, de la phase organique, l'eau résiduelle qui est restée après le lavage, de préférence par distillation azéotropique,
- ajouter la base organique et de préférence successivement l'aldéhyde à la solution de l'indanone, qui, de préférence, est libérée d'eau résiduelle, de sorte qu'un mélange de réaction est formé dans lequel l'indanone de formule (II) réagit avec l'aldéhyde aromatique de formule (III),
- éliminer l'eau formée durant la réaction, dudit mélange de réaction par distillation azéotropique,
- neutraliser le mélange de produit qui est présent après que la réaction s'est produite,
- distiller du solvant, dudit mélange de produit neutralisé,
- de préférence, distiller le produit,
- ensuite, de préférence, recristalliser le produit.

12. Procédé selon l'une quelconque des revendications précédentes, pour la préparation d'un composé de formule (I) ou d'un mélange comprenant un composé de formule (I),
les conditions du procédé sont ajustées de telle manière que le rapport molaire de
- la quantité produite du composé de formule (I) à
- la quantité du composé de formule (IV), produite, le cas échéant, comme sous-produit, 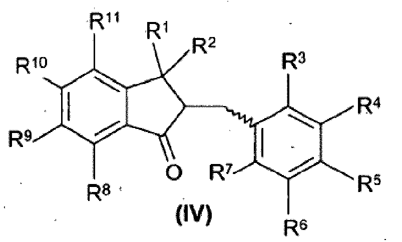 dans laquelle la signification des groupes R¹, R², R³, R⁴, R⁵, R⁶, R⁷, R⁸, R⁹, R¹⁰, R¹¹ et R¹² est la même que la signification des groupes désignés de façon égale dans le composé de formule (I),
est supérieur à 50, de préférence supérieur à 100, de manière préférée supérieur à 1000.

13. Mélange comprenant:
- un composé de formule (I), tel que défini dans l'une quelconque des revendications précédentes,
- le cas échéant un composé correspondant de formule (IV), tel que défini dans la revendication 12,
dans lequel le rapport molaire de la quantité totale du composé de formule (I) à la quantité totale, présente éventuellement, du composé de formule (IV) est supérieur à 50, de préférence supérieur à 100, de manière préférée supérieur à 1000,
- de préférence une substance donnant une couleur jaune audit mélange,
- une quantité de solvant choisi dans le groupe des solvants qui forment avec l'eau un azéotrope et qui, à 20 °C, présente une très mauvaise solubilité dans l'eau, le solvant formant avec l'eau un azéotrope possédant une solubilité dans l'eau qui, à 20 °C, est inférieure à 20 g de solvant par litre d'eau,
- ainsi que, le cas échéant, d'autres ingrédients,
apte à être produit selon un procédé selon l'une quelconque des revendications précédentes.

14. Mélange selon la revendication 13, le mélange étant une formulation cosmétique ou pharmaceutique.

15. Mélange selon la revendication 13 ou 14, pour l'application comme agent protecteur de la peau, agent photoprotecteur et/ou antagoniste de l'AhR.
